# EUROPEAN PATENT SPECIFICATION

(11) **EP 0 236 059 B1**
(45) Date of publication and mention of the grant of the patent: **27.04.1994**
(21) Application number: 87301626.5
(22) Date of filing: 25.02.1987
(51) Int. Cl.: C12N 15/27, C12P 21/02

(54) **Preparation of erythropoietin-producing cells and production process of erythropoietin using same**
Herstellung von erythropoietinproduzierenden Zellen und Verfahren zur Herstellung von Erythropoietin mit Verwendung dieser Zellen
Préparation des cellules produisant de l'erythropoiétine et procédé de production de l'erythropoiétine à partir de ces cellules

(30) Priority: 27.02.1986 JP 42275/86; 19.05.1986 JP 112537/86
(43) Date of publication of application: 09.09.1987
(73) Proprietor: SNOW BRAND MILK PRODUCTS CO. LTD., Sapporo-shi Hokkaido (JP)
(72) Inventor: Ueda, Masatsugu, Kawagoe-shi Saitama-ken (JP); Akai, Kunihisa, Utsunomiya-shi Tochigi-ken (JP); Murakami, Akihiko, Utsunomiya-shi Tochigi-ken (JP); Chiba, Hideo, Uji-shi Kyoto-fu (JP); Sasaki, Ryuzo, Kyoto-shi Kyoto-fu (JP)
(74) Representative: Hughes, Brian Patrick

(56) References cited:
- EP-A- 0 148 605
- EP-A- 0 148 605
- WO-A-86/03520
- WO-A-86/03520
- US-A- 4 465 624
- PROCEEDINGS OF THE NATIONAL ACADEMY OF SCIENCES OF THE USA, vol. 83, no. 17, September 1986, pages 6465-6469, Washington, DC, US; J.S. POWELL et al.: "Humanerythropoietin gene: High level expression in stably transfected mammaliancells and chromosome localization"
- PROCEEDINGS OF THE NATIONAL ACADEMY OF SCIENCES OF THE USA, vol. 83, no. 2, January 1986, pages 409-413, Washington, US; F.D. LEDLEY et al.: "Retroviral-mediated gene transfer of human phenylalanine hydroxylase into NIH 3T3 andhepatoma cells"
- CHEMICAL ABSTRACTS, vol. 106, no. 3, 19th January 1987, page 206, abstract no.14194k, Columbus, Ohio, US; Z. JIN: "Recombination of HBsAg gene and analysisof restriction map of the constracted plasmid DNA"
- CHEMICAL ABSTRACTS, vol. 106, no. 3, 19th January 1987, page 207, abstract no. 14207s, Columbus, Ohio, US; Z. JIN et al.

## Description

### Background of the Invention:

This invention relates to the preparation of animal cells capable of producing human erythropoietin which is an erythropoietic factor or an accelerating factor of erythrocyte-formation and to a process for the efficient production of human erythropoietin by culturing the cells thus-prepared.

### Description of the Prior Art:

Erythropoietin is an accelerating factor of erythrocyte-formation which acts on the erythroid precursor cells (CFU-E) present in the bone marrow to accelerate the differentiation thereof into erythrocyte and the properties of human erythropoietin are reported as described below.

It has been reported separately that human erythropoietin is a glycoprotein having a molecular weight of 35,000 [Yanagawa, S. et al. J. Biol. Chem. 259, 2707 - 2710 (1984)] and the peptide moiety thereof is a single-stranded polypeptide consisting of 166 amino acids [Jacob, K. et al. Nature 313, 806 - 810 (1985)].

Further, the structures of cDNA and genome DNA of human erythropoietin have been elucidated, as seen in the paper of Jacob, K. et al.

As regards the clinical effects of erythropoietin, stimulating effects of erythropoietin on the formation of erythrocyte have been recognized on the basis of animal experiments using purified erythropoietin preparations taken from the urine of anemic patients (Masunaga, H. et al. Acta Hematol Jpn. in press).

Accordingly, erythropoietin may possibly find clinical applications as medicines adaptable to the treatment of anemia of renal patients, to the prevention of anemia of the patients receiving homodialysis because of renal insufficiency or after nephrectomy and to the promotion of recovery of postoperative patients by stimulating the formation of erythrocyte.

It has thus been expected, as described above, to apply erythropoietin to the treatment of anemia from a clinical view point. However, it is the existing state of art that erythropoietin is backward in its development as a medicine due to the difficulty in providing a medically pure product in large amounts. Being contained in the urine of patients with aplastic anemia, erythropoietin separated from the urine and purified has been used to date only for the purpose of tests and research.

The present invention has been completed with the aim of solving the aforementioned problems in the production of erythropoietin. It is thus an object of the present invention to provide a process for the preparation of erythropoietin-producing cells capable of producing erythropoietin at a high level by utilizing a recombinant DNA technique and a process for the production of pure erythropoietin in high yields and large amounts which comprises culturing the cells formed in the above manner to produce erythropoietin and purifying the resultant erythropoietin by the use of a monoclonal antibody against human erythropoietin.

According to the present invention, there is provided a process for the production of erythropoietin which comprises the steps of; (1) preparing an erythropoietin expression vector in which erythropoietin expression is under the control of the M-MuLV LTR (Moloney murine leukemia virus) promoter, (2) introducing said erythropoietin expression vector into BHK21 cells, (3) cloning the cells to establish a cell-line constitutively producing erythropoietin, (4) culturing said cell line to produce erythropoietin and (5) isolating the erythropoietin.

The invention will now be explained in more detail by way of example in the following description.

The erythropoietin-producing cells in accordance with the present invention are prepared in the following manner:
First of all, an erythropoietin-expression vector is constructed by the following procedures.

### (1) Preparation of an erythropoietin-expression vector containing selective marker neo^{r} gene:

A plasmid phEP1404, which is formed by inserting an entire erythropoietin gene obtained from Human Genome DNA Library into a plasmid pUC8 at the cleavage sites for restriction endonucleases EcoRI and SmaI, is cleaved with restriction endonucleases BglII and BamHI to separate an erythropoietin gene. The erythropoietin gene is introduced into a shuttle vector pZIP-NeoSV(X)1 for mammalian cells at the restriction endonuclease BamHI cleavage site located on the downstream of LTR and upstream of the neo^{r} gene. A vector in which the erythropoietin gene is inserted correctly is selected as the intended vector according to the restriction endonuclease mapping.

The present invention permits the preparation of cells capable of expressing an erythropoietin gene at a high level by way of introduction of the erythropoietin gene in animal cells by using the erythropoietin-expression vectors prepared in the manner as described above (1). The erythropoietin gene is introduced in animal cells by the following procedure:

### Introduction of erythropoietin gene in animal cells by the use of the above vectors:

In the case of using an erythropoietin-expression vector containing selective marker neo^{r} gene prepared by the above procedure (1), the vector alone is introduced into BHK21 cells (originating from kidney of baby Silian hamster) with the calcium phosphate DNA transfection method.

The expression of the erythropoietin gene in the animal cells by way of the introduction of the erythropoietin gene thereinto as described above can be confirmed by the formation of G418-resistant cells. Specifically, this can be done by diluting the cells having the erythropoietin gene introduced, inoculating the cells to a culture medium followed by incubation, adding G418 to the culture medium followed by incubation, selecting G418-resistant cells thus-formed and then selecting among them cells producing erythropoietin to the culture medium.

The cells thus-selected are subjected to limiting dilution to clone the cells expressing the erythropoietin gene, thereby selecting cells capable of expressing the gene at a high level. Thus, a cell strain constitutively producing erythropoietin is established.

Identification of the erythropoietin produced from the strain thus-established is made in accordance with the radioimmunoassay method and the in-virto bioassay method using mouse fetal liver cells.

### Production of erythropoietin using erythropoietin-producing cells:

The erythropoietin-producing cells prepared as described above are cultured in a serum-containing synthetic medium and the supernatant of the resulting culture medium is subjected to ultrafiltration (nominal molecular cut off of 13,000) to concentrate and separate high molecular components. Thereafter, the supernatant is allowed to pass through a column containing a monoclonal antibody against human erythropoietin. The eluate obtained from the subsequent elution is gel-filtered to isolate erythropoietin, thus obtaining purified recombinant erythropoietin.

The present invention will be described specifically with reference to the following examples. It is however to be understood that the scope of the present invention is not limited to or by these examples.

### Example 1:

This example illustrates the production of erythropoietin by erythropoietin-producing cells prepared using a vector containing a selective marker gene and an erythropoietin gene on the same vector.

### (i) Preparation of an erythropoietin-expression vector containing a selective marker neo^{r} gene (pZIPNeoSV(X)1EPO):

To 10 µg of a plasmid phEP1404 (a plasmid formed by inserting a fragment formed by cleaving an erythropoietin gene at the position 2.4 kb downstream from the Apat cleavage site at the 5' end into a plasmid pUC8 at the cleavage sites of restriction endonucleases EcoRI and SamI; size 5.1 kb) dissolved in 116 µl of TE-buffer solution (10 mM Tris-HCl, 1 mM EDTA, pH 7.4) were added 40 µl of a BglII reaction buffer solution of fivefold concentration (50 mM Tris-HCl, 35 mM MgCl₂, 500 mM NaCl, 35 mM 2-mercaptoethanol), followed by addition of 20 units each of restriction endonucleases BglII and BamHI. Having undergone reaction at 37°C for 2 hours, the reaction mixture was subjected to 3.5% acrylamide gel electrophoresis to cut out a gel strip corresponding to the BglII-BamHI fragment of 2.4 kb (erythropoietin gene). After being ground minutely, the gel was added with 1.0 ml of an elution buffer solution (0.5 M ammonium acetate, 1 mM EDTA, 0.1% SDS, pH 8.0) and incubated at 37°C overnight to submit it to DNA extraction. The DNA containing solution was centrifuged to precipitate the acrylamide gel, and the supernatant was collected and treated by extraction with phenol once and extraction with ether three times, thereby removing phenol contained in the water phase. The resulting water phase was added with a twofold amount of ethanol to precipitate a DNA fragment. The DNA fragment (precipitate) was recovered by centrifugation, dried and dissolved in 20 µl of sterilized water, thereby preparing an erythropoietin gene solution.

Separately, 20 µl of a BamHI reaction buffer solution of fivefold concentration (same as the BglII reaction buffer solution) and then a restriction endonuclease BamHI were added to 5 µg of a shuttle vector pZIP NeoSV(X)1(dissolved in 5 µl of TE-buffer solution), followed by reaction at 37°C for one hour, thereby cleaving the vector.

The reaction solution was treated by extraction with phenol once, extraction with ether three times and precipitation with ethanol once and the precipitated DNA was dried. To 350 ng (nanogram: 10⁻⁹ gram) of the BamHI-cleaved pZIP-NeoSV(X)1 (dissolved in 4 µl of water) were added 200 ng of the erythropoietin gene (16 µl), 2 µl of a ligation reaction buffer solution of tenfold concentration (660 mM Tris-HCl, 66 mM MgCl₂, 100 mM DTT, pH 7.6), 2 µl of 9 mM ATP and 3 µl of T₄ DNA ligase (8.4 units), followed by reaction at 4°C overnight. The reaction solution was treated by extraction with phenol twice, extraction with ether three times and precipitation with ethanol once. The resulting DNA was dried and dissolved in 20 µl of TE-buffer solution, thereby preparing a transducing DNA solution. Using 10 µl of the DNA solution, 200 µl of component cells of Escherichia coli DH-1 were transformed (transformation frequency: 3 x 10⁶ cells µg pBR322).

By the above procedure, 35 clones of ampicillin-kanamycin-resistant transformant were obtained. Of these, 11 clones were analyzed by restriction endonuclease mapping of plasmid to screen 4 containing the intended erythropoietin-expression vector. Using one clone of them, a plasmid preparation was carried out in a usual manner to prepare an erythropoietin-expression vector (pZIPNeoSV(X)1EPO). Using the erythropoietin-expression vector thus-prepared, an erythropoietin gene was introduced into animal cells in accordance with the DNA transfection method in the following manner.

### (ii) Introduction of an erythropoietin gene into BHK21 cells:

2 x 10⁶ BHK21 cells were seeded in a 25 cm² T-flask [culture medium: Basal Medium Eagle (BME) + 10%CS + 10%Tryptose Phosphate Broth] the day before DNA transfection. Using the DNA-calcium phosphate solution as described above, DNA transfection into BHK21 cells was conducted. After selecting G418-resistant cells, they were subjected to limiting dilution twice to clone the cells, thereby establishing a cell line constitutively producing erythropoietin, named EPOBX7A. Erythropoietin produced by the cell line and its production amount were assayed in accordance with the radioimmunoassay method and the in vitro bioassay method using mouse fetal liver cells. The activities measured with the both methods were the same 1,000 units/10⁶ cells/day.

The erythropoietin-producing cells EPOBX7A prepared as described above were cultured in the below-described manner, thereby producing erythropoietin followed by its isolation.

### (iii) Production and isolation of recombinant erythropoietin:

Into cellfactory 10 chamber (6,000 cm²) (product of Nunc Co.) were seeded 1.5 x 10⁸ cells of EPOBX7A, which were then cultured for 3 days in the presence of 1 ℓ of the culture medium [Dulbecco's Modified Eagle MEM(DME) + 10% calf serum (CS)]. The culture medium was replaced freshly by 2 ℓ of the culture medium every 3 days. Erythropoietin activity contained in the culture medium was determined in accordance with the radioimmunoassay method and found to be 100 - 300 units/ml. Isolation of erythropoietin was effected in accordance with the method described in Japanese Patent Laid-Open No. 41614/1985.

Then, 11 l of the culture medium (having an EPO activity of 2 x 10⁶ units) obtained in the above manner were treated with a ultrafiltration apparatus to obtain a concentrated fraction of a molecular weight of 10,000 or more while controlling the temperature (at 5 - 10°C), added with PBS (phosphate buffer saline) and further concentrated, thereby obtaining 1.5 l of an erythropoietin concentrate.

The concentrate was added with a SDS powder to a concentration of 2% and heated at 100°C for 3 minutes. The mixture was cooled to 4°C and allowed to stand at 0°C for overnight, followed by centrifugation to remove SDS, thus obtaining 1.2 l of a supernatant. The supernatant was passed through an immunoaffinity column prepared by coupling a monoclonal antibody against human erythropoietin onto Affi-Gel 10 (amount of antibody coupled: 0.5 g/10 ml Affi-Gel 10; 3.6 cm x 3 cm, bed volume 30 ml) at a rate of 60 ml/hr. Then, 2,000 ml of PBS, 400 ml of a 10 mM phosphate buffer solution containing 0.5 M NaCl (pH 7.4) and 400 ml of 0.15 M NaCl were passed through the column in this order at a rate of 100 ml/hr to wash the column. Thereafter, a mixed solution of 0.2 M acetic acid and 0.15 M NaCl was allowed to flow therethrough at a rate of 30 ml/hr to obtain 100 ml of an eluate. The eluate contained 1.2 x 10⁶ units of erythropoietin activity. Then, a gel-filtration was conducted in the following manner.

To the eluate was added a 3.4 M Tris solution properly, followed by neutralization. Having undergone dialysis against water, the resulting solution was concentrated by freeze-drying. The freeze-dried powder was dissolved in 6 ml of a 10 mM sodium phosphate buffer solution containing 0.15 m NaCl (pH 7.5), and 3 ml of the solution were passed through a Sephadex G100-packing column (1.2 cm x 150 cm, bed volume: 170 ml) equilibrated in advance with the same buffer solution as described above at a rate of 6 ml/hr to fractionate the eluate according to molecular weight. By removing fractions containing high molecular impurities, an erythropoietin-active fraction was obtained. Similarly, 3 ml of the remaining erythropoietin concentrate were subjected to molecular weight fractionation to obtain another erythropoietin-active fraction. The erythropoietin obtained by the above procedures had an activity of 1.08 x 10⁶ units. Purity of the preparations thus-obtained was assayed in accordance with the SDS polyacrylamide electrophoresis method. However, no impure proteins were detected in the preparations (a single band was observed at the portion corresponding to a molecular weight of about 35,000).

Thus, recombinant erythropoietin of high purity was obtained.

### (iv) Preparation of an erythropoietin cDNA-transducing vector containing a selective marker neo^{r} gene (pZIPNeoSV(X)Epo cDNA):

To 20 µg of the plasmid containing the above erythropoietin cDNA dissolved in 70 µl of TE-buffer solution were added 20 µl of a Sau3AI reaction buffer solution of 5-fold concentration (10 mM Tris HCl pH 7.5, 7 mM MgCl₂, 100 mM NaCl) and then 50 units of a restriction endonuclease Sau3AI (10 µl), followed by reaction at 37°C for 2 hours. The reaction solution was subjected to 3.5% acrylamide gel electrophoresis to cut out a gel strip corresponding to a 800 by fragment (erythropoietin cDNA). The DNA was extracted from the gel and dissolved in 20 µl of sterilized water to prepare an erythropoietin cDNA solution.

To 180 ng of the erythropoietin cDNA were added 330 ng of the BamHI-cleaved PZIPNeoSV(X)1, followed by precipitation with ethanol and drying of the DNA. To the dried DNA were added 1 µl of a ligation reaction buffer solution of 10-fold concentration, 1 µl of 9 mM ATP and 1 µl of T₄ DNA ligase (2.8 units), followed by reaction at 4°C overnight. The reaction solution was treated by extraction with phenol twice, extraction with ether three times and precipitation with ethanol once. The precipitated DNA was dried and dissolved in 10 µl of TE-buffer solution to prepare a transducing DNA solution. Using 5 µl of the DNA solution, 200 µl of competent cells of Escherichia coli DH-1 were transformed.

By the above procedure, 52 clones of ampicillin-kanamycin-resistant transformant were obtained. Of these, 15 clones were subjected to the restriction endonuclease mapping of plasmid to select one clone containing the intended erythropoietin cDNA-transducing vector. Using this clone, a plasmid preparation was carried out in a usual manner, thus preparing an erythropoietin cDNA-transducing vector(pZIPNeoSV(X)EpocDNA).

### Introduction of erythropoietin cDNA into BHK21 cells:

To 25 cm²-T flasks were seeded 2 x 10⁵ cells of BHK21. The following day they were subjected to DNA transfection according to the calcium phosphate method. In 50 µl of water were dissolved 25 µg of pZIP NeoSV(X)EpocDNA which has been previously cleaved with a restriction endonuclease SalI, followed by mixing with 75 µl of 2 M calcium chloride solution and 525 µl of water to prepare a solution A. Then, a solution B was prepared by mixing 625 µl of 50 mM HEPES containing 0.28 M NaCl (pH 7.1) and 25 µl of 35 mM NaH₂PO₄-35 mM Na₂HPO₄. While stirring the solution B vigorously, the solution A was added dropwise thereto slowly, thereby co-precipitating DNA with calcium phosphate. The precipitate was allowed to grow by letting it stand at room temperature for 30 minutes.

To 4 ml each of the culture media (DME-10%CS) of the previously prepared BHK21 cells was added 0.3 ml of the above-prepared DNA-calcium phosphate solution, and the cells were cultured in a CO₂ incubator for 18 hours.

The resulting culture media were added with 0.6 ml of a PBS solution containing 15% glycerol and allowed to stand for 2 minutes. The cells were then washed with a DME three times, added individually with 4 ml of the above culture medium and cultured again in the CO₂ incubator. One day later, the culture media were exchanged. The following day, the cells were transferred separately to two 9 cm diameter schales for seeding (1/5 Split). Then, the next day, the culture media were replaced by culture media containing 400 µl/ml of G418. Thereafter, the cells were cultured for 2 weeks while exchanging the culture media from time to time, thus selecting G418-resistant cells and cloning them according to the limiting dilution method. The cloning was repeated once to establish cell line BXC constitutively producing erythropoietin. The amounts of erythropoietin produced from the cell strains were the same 100 units/10⁶ cells/day according to the radioimmunoassay method.

## Claims

1. A process for the production of erythropoietin which comprises the steps of; (1) preparing an erythropoietin expression vector in which erythropoietin expression is under the control of the M-MuLV LTR (Moloney murine leukemia virus) promoter, (2) introducing said erythropoietin expression vector into BHK21 cells, (3) cloning the cells to establish a cell-line constitutively producing erythropoietin, (4) culturing said cell line to produce erythropoietin and (5) isolating the erythropoietin.

2. A process according to claim 1 in which the erythropoietin expression vector is pZIPNeoSV(X)1EPO (as hereinbefore described in Example 1(i)

## Patentansprüche

1. Verfahren zur Herstellung von Erythropoietin, das die folgenden Schritte umfaßt: (i) einen Erythropoietin Expressionsvektor, in dem die Expression des Erythropoietin unter der Kontrolle des M-MuLV LTR (Moloney murine leukemia virus) Promotors steht, herzustellen, (ii) diesen Erythropoietin Expressionsvektor in BHK21 Zellen einzuführen, (iii) die Zellen zu klonieren, um eine Zellinie zu etablieren, die Erythropoietin konstitutiv herstellt, (iv) diese Zellinie zur Produktion von Erythropoietin zu kultivieren und (v) das Erythropoietin zu isolieren.

2. Das Verfahren nach Anspruch 1, wobei der Erythropoietin Expressionsvektor pZIP NeoSV(X)1EPO ist (wie hierin zuvor in Beispiel 1 (i) beschrieben).

## Revendications

1. Procédé de production d'érythropoïétine qui comprend les étapes selon lesquelles (1) on prépare un vecteur d'expression d'érythropoïétine dans lequel l'expression de l'érythropoïétine est sous le contrôle du promoteur de M-MuLV LTR (virus des leucémies de la souris de Moloney), (2) on introduit ledit vecteur d'expression d'érythropoïétine dans des cellules BHK21, (3) on clone les cellules pour établir une lignée cellulaire productrice de façon constitutive d'érythropoïétine, (4) on cultive ladite lignée cellulaire pour produire de l'érythropoïétine et (5) on isole l'érythropoïétine.

2. Procédé selon la revendication 1, dans lequel le vecteur d'expression d'érythropoïétine est pZIPNeoSV(X)1EPO (tel que décrit précédemment dans l'exemple 1(i)).
